(19) Europäisches Patentamt
European Patent Office
Office européen des brevets

(11) Veröffentlichungsnummer: **0 109 930**
**B1**

(12) # EUROPÄISCHE PATENTSCHRIFT

(45) Veröffentlichungstag der Patentschrift: **17.09.86**

(21) Anmeldenummer: **83810481.8**

(22) Anmeldetag: **19.10.83**

(51) Int. Cl.⁴: **C 07 D 239/91**, C 07 D 403/04, B 41 M 5/16, B 41 M 5/18, B 01 J 13/02

(54) **Chromogene Bis-Chinazolinverbindungen, Verfahren zu ihrer Herstellung und ihre Verwendung als Farbbildner in druckempfindlichen oder wärmeempfindlichen Aufzeichnungsmaterialien.**

(30) Priorität: **25.10.82 CH 6201/82**
**28.06.83 CH 3523/83**

(43) Veröffentlichungstag der Anmeldung: **30.05.84 Patentblatt 84/22**

(45) Bekanntmachung des Hinweises auf die Patenterteilung: **17.09.86 Patentblatt 86/38**

(84) Benannte Vertragsstaaten: **BE CH DE FR GB IT LI**

(56) Entgegenhaltungen:
**EP - A - 0 033 716**
**DE - A - 2 020 297**
**DE - A - 2 644 265**
**DE - B - 1 251 348**
**GB - A - 1 355 124**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Zink, Rudolf, Alemannenstrasse 2, CH-4106 Therwil (CH)**
Erfinder: **Fletcher, Ian John, Dr., Bürgenstal 27, CH-4312 Magden (CH)**

**EP 0 109 930 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft chromogene Bis-Chinazolinverbindungen, Verfahren zu ihrer Herstellung und ihre Verwendung als Farbbildner in druckempfindlichen oder wärmeempfindlichen Aufzeichnungsmaterialien.

Aus der EP-A-0033716 sind chromogene Chinazolinverbindungen bekannt, die in 2-Stellung durch eine 4-Aminophenylgruppe und in 4-Stellung durch eine Aryloxygruppe substituiert sind.

Die erfindungsgemässen Bis-Chinazolinverbindungen entsprechen der allgemeinen Formel (1)

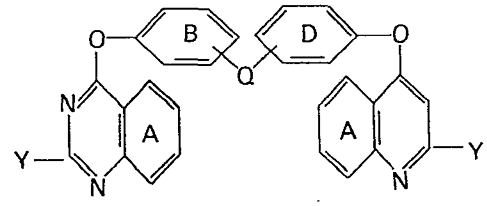

worin

die Ringe A, B und D, unabhängig voneinander, unsubstituiert oder durch Cyano, Nitro, Halogen, Niederalkyl, Phenyl, Benzyl, Niederalkoxy oder Niederalkoxycarbonyl substituiert sein können,

Q die direkte Bindung, einen aliphatischen oder cycloaliphatischen Kohlenwasserstoffrest mit höchstens 8 Kohlenstoffatomen, -CO-, -S- oder $-SO_2-$, und

Y einen Aminophenylrest der Formel

(2a) 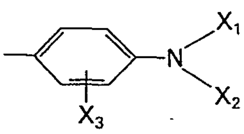 oder

einen hydrierten heterocyclischen Rest der Formel

(2b) 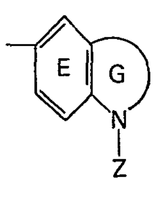

bedeuten, wobei

$X_1$ und $X_2$, unabhängig voneinander, je Wasserstoff, unsubstituiertes oder durch Halogen, Hydroxy, Cyano oder Niederalkoxy substituiertes Alkyl mit höchstens 12 Kohlenstoffatomen, Cycloalkyl, Phenyl, Benzyl oder durch Halogen, Nitro, Cyano, Niederalkyl, Niederalkoxy oder Niederalkoxycarbonyl substituiertes Phenyl oder Benzyl

oder $X_1$ und $X_2$ zusammen mit dem sie verbindenden Stickstoffatom einen fünf- oder sechsgliedrigen, vorzugsweise gesättigten, heterocyclischen Rest und

$X_3$ Wasserstoff, Halogen, Nitro, Niederalkyl, Niederalkoxy oder Niederalkoxycarbonyl und

Z Wasserstoff oder unsubstituiertes oder durch Halogen, Cyano oder Niederalkoxy substituiertes Alkyl mit höchstens 8 Kohlenstoffatomen, Cycloalkyl oder Benzyl und wobei der

Ring E unsubstituiert oder durch Cyano, Halogen, Niederalkyl z.B. Methyl oder Niederalkoxy wie Methoxy substituiert sein kann und der Ring G einen hydrierten fünf- oder sechsgliedrigen Stickstoffheterocyclus darstellt, der gegebenenfalls als Ringglied ein weiteres Heteroatom, z.B. Sauerstoff, Schwefel oder Stickstoff aufweist und unsubstituiert oder durch Halogen, Cyano, Hydroxyl, Niederalkyl, Niederalkoxy, $C_5$–$C_6$-Cycloalkyl, Benzyl oder $C_3$–$C_6$-Alkylen einfach oder je nach Substituenten mehrfach C-substituiert ist, wobei die Bezeichnung Nieder $C_1$–$C_5$- darstellt.

Niederalkyl und Niederalkoxy stellen bei der Definition der Reste der Bis-Chinazolinverbindungen solche Gruppen oder Gruppenbestandteile dar, die 1 bis 5, vorzugsweise 1 bis 3 Kohlenstoffatome aufweisen, wie z.B. Methyl, Ethyl, n-Propyl, Isopropyl, n-Butyl, sek.-Butyl, tert.-Butyl oder Amyl bzw. Methoxy, Ethoxy, Isopropoxy oder tert.-Butoxy.

Q befindet sich zweckmässigerweise in para-Stellung zu beiden Sauerstoffatomen.

Q stellt in der Bedeutung eines aliphatischen Kohlenwasserstoffrestes insbesondere eine Alkylen- oder Alkylidengurppe dar. Diese können bis zu 8 Kohlenstoffatomen enthalten und geradkettig oder verzweigt sein. Die Alkylengruppe weist vorzugsweise 1 bis 4 Kohlenstoffatome auf. Es handelt sich beispielsweise um die $-CH_2-$, $-CH_2CH_2-$, $-CH_2CH_2CH_2-$, $-CH_2CH(CH_3)-CH_2-$ oder $-CH_2CH_2CH_2CH_2-$ Gruppe. Die Alkylidengruppe ist vorzugsweise Ethyliden, Propyliden, Isopropyliden, Butyliden oder sek.-Butyliden. Als cycloaliphatischer Kohlenwasserstoffrest bedeutet Q z.B. die Cyclopentylengruppe, die Cyclohexylengruppe, die Cyclopentylidengruppe oder vor allem die Cyclohexylidengruppe. Diese cycloaliphatischen Reste können eine oder zwei Methylgruppen aufweisen.

Vorzugsweise bedeutet Q den aliphatischen oder cycloaliphatischen Rest, insbesondere Methylen oder Alkyliden mit höchstens 4 Kohlenstoffatomen und vor allem Isopropyliden oder Butyliden.

Die Ringe A, B und D sind vorzugsweise nicht weiter substituiert. Falls sie Substituenten aufweisen, sind sie unabhängig voneinander in erster Linie durch Halogen, Niederalkyl oder Niederalkoxy, z.B. durch Chlor, Methyl, Isopropxyl, tert.-Butyl oder Methoxy weitersubstituiert. Pro Benzolring können vorteilhafterweise 1 oder 2 Substituenten vorhanden sein. Bevorzugte Substituenten der Benzolringe B und D sind auch Methyl und tert.-Butyl sowie Phenyl oder Benzyl.

Stellen die Substituenten $X_1$ und $X_2$ Alkylgruppen dar, so können sie geradkettig oder verzweigt sein. Beispiele für solche Alkylreste sind Methyl, Ethyl, n-Propyl, Isopropyl, n-Butyl, sek.-Butyl, Amyl, n-Hexyl, 2-Ethyl-hexyl, n-Octyl, Isooctyl oder n-Dodecyl.

Sind die Alkylreste in $X_1$ und $X_2$ substituiert, so handelt es sich vor allem um Cyanoalkyl, Halogenalkyl, Hydroxyalkyl oder Alkoxyalkyl mit insgesamt vorzugsweise 2 bis 4 Kohlenstoffatomen, wie z.B. β-Cyanoethyl, β-Chloroethyl, β-Hydroxyethyl, β-Methoxyethyl oder β-Ethoxyethyl.

Beispiele für Cycloalkyl in der Bedeutung von $X_1$ und $X_2$ sind Cyclopentyl oder vorzugsweise Cyclohexyl.

Bevorzugte Substituenten in der Benzyl- und Phenylgruppe von $X_1$ und $X_2$ sind z.B. Halogen, Cyano, Methyl, Methoxy oder Carbomethoxy. Beispiele für derartige araliphatische bzw. aromatische Reste sind Methylbenzyl, Chlorbenzyl, Cyanophenyl, Tolyl, Xylyl, Chlorphenyl, Methoxyphenyl oder Carbomethoxyphenyl.

Wenn $X_1$ und $X_2$ zusammen mit dem gemeinsamen Stickstoffatom einen heterocyclischen Rest darstellen, so ist dieser beispielsweise Pyrrolidino, Piperidino, Pipercolino, Morpholino, Thiomorpholino oder Piperazino, wie z.B. N-Methylpiperazino. Bevorzugte heterocyclische Reste für $NX_1X_2$ sind Pyrrolidino, Piperidino oder Morpholino.

$X_1$ und $X_2$ bedeuten, unabhängig voneinander, vorzugsweise Niederalkyl, Benzyl, Phenyl, Niederalkylphenyl oder Niederalkoxyphenyl. $X_3$ bedeutet vorzugsweise Wasserstoff, Chlor, Methyl, Methoxy oder Ethoxy.

Der Ring E ist vorzugsweise unsubstituiert. Er kann jedoch vorteilhafterweise eine Methylgruppe aufweisen. Der Ring G ist vorzugsweise sechsgliedrig und vor allem durch 1, 2 oder 3 Methylgruppen C-substituiert.

Z ist vorzugsweise Niederalkyl, Benzyl oder β-Cyanoethyl.

Unter den Bis-Chinazolinverbindungen der Formel (2) sind diejenigen, in denen $Y_1$ einen Rest der Formel (2a) darstellt, bevorzugt.

Praktisch wichtige chromogene Bis-Chinazolinverbindungen entsprechen der Formel

(3) 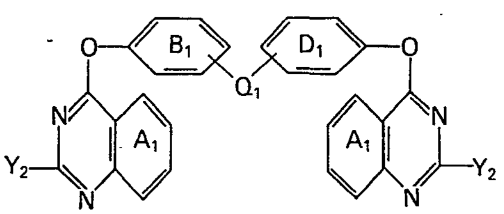

worin

$Q_1$ die direkte Bindung, einen geradkettigen oder verzweigten Alkylen- oder Alkylidenrest je mit höchstens 8, vorzugsweise höchstens 4 Kohlenstoffatomen oder den Cyclohexylidenrest

$Y_2$ einen Aminophenylrest der Formel

(3a) 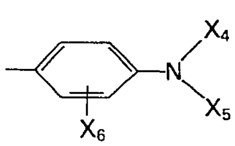

einen 5-Indolinrest der Formel

(3b) 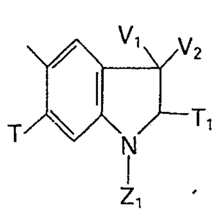

einen Tetrahydrochinolinylrest der Formel

(3c) 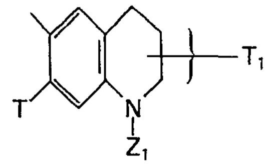

einen Tetrahydrochinolinylrest der Formel

(3d) 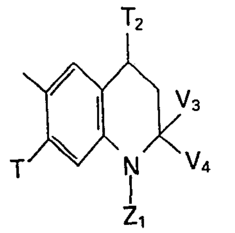

oder einen Benzomorpholinorest der Formel

(3e) 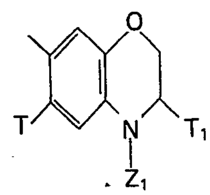

bedeuten, wobei

$X_4$ und $X_5$, unabhängig voneinander, Niederalkyl, Cyano-Niederalkyl, Benzyl, Phenyl, Niederalkylphenyl oder Niederalkoxyphenyl oder

$X_4$ und $X_5$ zusammen mit dem sie verbindenden Stickstoffatom Pyrrolidino, Piperidino oder Morpholino,

$X_6$ Wasserstoff, Halogen, Niederalkyl oder Niederalkoxy,

$Z_1$ Wasserstoff, $C_1$–$C_8$-Alkyl, $C_2$–$C_6$-Akoxyalkyl, β-Cyanoethyl oder Benzyl

T Wasserstoff, Halogen, Niederalkyl oder Niederalkoxy,

$T_1$ und $T_2$ je Wasserstoff, Halogen, Hydroxy, Niederalkyl oder Niederalkoxy und

$V_1$, $V_2$, $V_3$ und $V_4$ je Wasserstoff, Niederalkyl, $C_5$–$C_6$-Cycloalkyl oder Benzyl oder ($V_1$ und $V_2$) oder ($V_3$ und $V_4$) je zusammen $C_3$–$C_6$-Alkylen bedeuten und

die Ringe $A_1$, $B_1$ und $D_1$, unabhängig voneinander, unsubstituiert oder durch einen oder zwei Substituenten ausgewählt aus Cyano, Halogen, Niederalkyl, Phenyl oder Niederalkoxy substituiert sein können.

Unter den Bis-Chinazolinverbindungen der Formel (3) sind diejenigen, in denen $Y_2$ einen Aminophenylrest der Formel (3a) darstellt, bevorzugt. Dabei sind $X_4$ und $X_5$ Niederalkyl oder Benzyl. $X_6$ ist vorzugsweise Wasserstoff. $Q_1$ ist vorzugsweise in p-Stellung zu den O-Atomen und stellt vorteilhafterweise Alkylen oder Alkyliden je mit höchstens 4 Kohlenstoffatomen, insbesondere Isopropyliden oder Butyliden dar. $Q_1$ ist bevorzugt auch der Cyclohexylidenrest. Der Ring $A_1$ ist vorzugsweise unsubstituiert. Die Benzolringe $B_1$ und $D_1$ sind vorzugsweise unsubstituiert oder durch Methyl und/oder tert.-Butyl substituiert.

Bei den Bis-Chinazolinverbindungen der Formel (3), in der $Y_2$ einen Rest der Formel (3b), (3c), (3d)

oder (3e) darstellt, ist der N-Substituent $Z_1$ insbesondere Benzyl, β-Cyanoethyl oder Alkyl mit 1 bis 8 Kohlenstoffatomen, z. B. n-Octyl, n-Butyl, Isopropyl oder vor allem Methyl oder Ethyl.

Dabei ist

$Y_2$ in erster Linie der Tetrahydrochinolinrest der Formel (3d).

T ist vorzugsweise Wasserstoff oder Methyl.

$T_1$ ist vorzugsweise Wasserstoff, Methyl, Hydroxyl oder Chlor.

$T_2$ ist vorzugsweise Wasserstoff, Methyl oder Ethyl.

$V_1$ und $V_2$ bedeuten vorzugsweise Wasserstoff oder Methyl.

$V_3$ und $V_4$ bedeuten vorzugsweise jeweils Niederalkyl und insbesondere jeweils Methyl.

Bedeuten ($V_1$ und $V_2$) oder ($V_3$ und $V_4$) zusammen Alkylen, so weisen sie mit Vorteil 4 oder 5 Kohlenstoffatome auf und bilden mit dem sie verbindenden Kohlenstoffatom einen Cyclopentan- oder Cyclohexanring.

Von grossem Interesse sind Bis-Chinazolinverbindungen der Formel

(4) 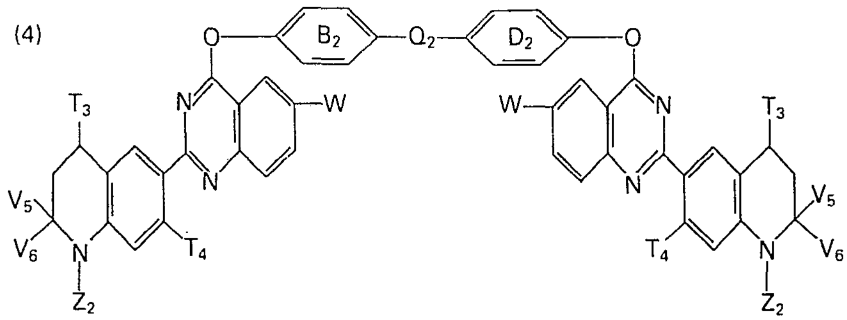

oder vor allem Bis-Chinazolinverbindungen der Formel

(5) 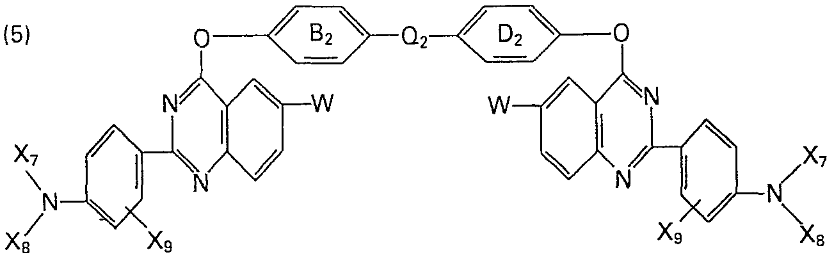

worin

$Q_2$ geradkettiges oder verzweigtes Alkylen oder Alkyliden je mit höchstens 4 Kohlenstoffatomen oder Cyclohexyliden,

$X_7$ und $X_8$ Niederalkyl, β-Cyanoethyl, Benzyl oder Phenyl, oder $-NX_7X_8$ Piperidino,

$X_9$ Wasserstoff, Methyl, Methoxy oder Ethoxy,

$Z_2$ Alkyl mit 1 bis 8 Kohlenstoffatomen, β-Cyanoethyl oder Benzyl,

$T_3$, $V_5$ und $V_6$ je Niederalkyl, insbesondere Methyl oder Ethyl,

$T_4$ Wasserstoff oder Methyl, und

W Halogen, Methyl, Methoxy oder besonders Wasserstoff bedeuten,

und die Ringe $B_2$ und $D_2$ unsubstituiert oder durch einen oder zwei Substituenten ausgewählt aus Methyl, Methoxy und tert.-Butyl substituiert sind.

Im Vordergrund des Interesses stehen Bis-Chinazolinverbindungen der Formel (5), in der $Q_2$ Butyliden oder vor allem Isopropyliden bedeutet. $X_7$ und $X_8$ sind vorzugsweise Benzyl oder vor allem Niederalkyl.

Halogen in Verbindungen mit den vorstehenden Substituenten in Formeln (1) bis (5) bedeutet beispielsweise Fluor, Brom oder vorzugsweise Chlor.

Die erfindungsgemässen Bis-Chinazolinverbindungen der Formel (1) werden dadurch hergestellt, dass man 1 Mol einer Bis-Phenolverbindung der Formel

(6) 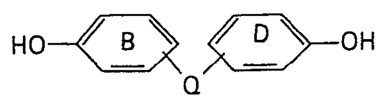

worin B, D und Q die angegebene Bedeutung haben, mit 2 Mol einer 4-Halogenchinazolinverbindung der Formel

(7) 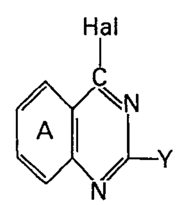

worin A und Y die angegebene Bedeutung haben und Hal Halogen, wie z. B. Brom, Fluor oder vorzugsweise Chlor bedeutet, umsetzt.

Die Umsetzung der Verbindung der Formel (6) mit der Verbindung der Formel (7) erfolgt zweckmässig in Anwesenheit eines säurebindenden Mittels, wie z. B. eines Alkalimetallhydroxides, Alkalimetallcarbonates, einer tertiären Stickstoffbase, wie z. B. Pyridin oder Trialkylaminen, und vorzugsweise in Gegenwart auch eines quaternären Ammoniumsalzes, wie z. B. Tetrabutylammoniumbromides, gegebenenfalls in einem organischen Lösungsmittel oder in einem wässrig-organischen zweiphasigen Medium und bei Rückflusstemperatur.

Als Lösungsmittel kommen beispielsweise cycloaliphatische oder aromatische Kohlenwasserstoffe, wie z.B. Cyclohexan, Benzol, Toluol oder Xylol; Chlorkohlenwasserstoffe, wie Chloroform, Ethylenchlorid oder Chlorbenzole; Ether, wie Diethylether oder Glykoldimethylether; cyclische Ether, wie Dioxan oder Tetrahydrofuran; sowie Dimethylformamid, Diethylformamid, Dimethylsulfoxid oder Acetonitril in Betracht.

Bisphenolverbindungen der Formel (6), die als Ausgangsstoffe für die Umsetzung mit den Chinazolinverbindungen der Formel (7) eingesetzt werden können, sind beispielsweise in der US-PS 3244550 beschrieben.

Als Beispiele für als Ausgangsstoffe der Formel (6) dienende Bisphenole seien genannt:

2,4'-Methylendiphenol, 4,4'-Methylendiphenol, 2,2'-Methylendiphenol,4,4'-(1",1"-Butyliden)-diphenol, 4,4'-sek.-Butylidendiphenol, 4,4'-Isopropylidendiphenol, 4,4'-Isopropyliden-bis-(2-methylphenol), 4,4'-Cyclopentylidendiphenol, 4,4'-Cyclohexylidendiphenol, 2,2'-Methylendi-p-cresol, 4,4'-Methylendi-o-cresol, 4,4'-Methylen-bis-(2-benzylphenol), 2,2'-Methylen-bis-(4-tert.-butylphenol), 2,2'-Methylen-bis-(4-tert.-pentylphenol), 2,2'-Methylen-bis-(4-chlorphenol), 4,4'-Methylen-bis-(2-chlorphenol), 4,4'-(1"-Methyl-n-hexyliden)-diphenol, 4,4'-(1",1"-Butyliden)-bis-(2-tert.-butyl-5-methylphenol), 2,2'-Methylen-bis-(4-phenol-phenol), 4,4'-Methylen-bis-(2-phenyl-phenol), 4,4'-Thiodiphenol, 4,4'-Sulphonyldiphenol, 2,2'-Diphenol, 3,3'-Diphenol, 4,4'-Diphenol, 4,4'-Dihydroxybenzophenon oder 2,2'-Dihydroxybenzophenon.

Die Ausgangsstoffe der Formel (7) können dadurch hergestellt werden, dass man beispielsweise ein 2-Amino-benzoesäureamid der Formel

(8) 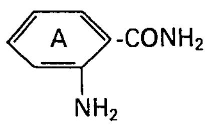

mit einem Aldehyd der Formel

(9) Y-CHO

zu einer 1,2,3,4-Tetrahydro-Chinazolon(4)-Verbindung der Formel

(10) 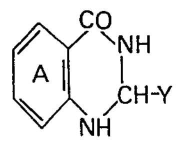

umsetzt, diese zu einer Verbindung der Formel

(11) 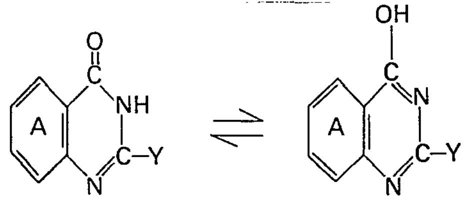

oxidiert, sodann die Hydroxylgruppe am heterocyclischen Ring des Chinazolinsystems durch ein Halogenatom, z.B. mittels Phosphoroxichlorid in Dichlorbenzol oder Thionylchlorid in Dimethylformamid unter Bildung des Ausgangsstoffes der Formel (7) ersetzt. Die erhaltene 4-Halogenchinazolinverbindung kann, ohne isoliert zu werden, weiterverwendet werden.

Die Oxidation der Umsetzungsprodukte der Formel (10) zu den 4-Chinazolon-verbindungen der Formel (11) erfolgt mit Oxidationsmitteln. Geeignete Oxidationsmittel sind z.B. Chromate, Bichromate, Chlorate, Chlorite, Peroxide, z.B. Wasserstoffperoxid, Mangandioxid, Bleidioxid, molekularer Sauerstoff, Luft, Perborate, Permanganate, Nitrite, Chlor, Brom und vor allem Chloranil oder Bisulfite.

Die besten Ergebnisse in bezug auf Ausbeute und Reinheit der erhaltenen 4-Chinazolon-verbindungen erzielt man mit Chloranil als bevorzugtes Oxidationsmittel.

Oekologische Vorteile bietet die Oxidation mit Natriumbisulfit. Auf analoge Weise wie in Synthesis 1981, (1), 35 beschrieben, erhält man unter Verwendung dieses Oxidationsmittels Chinazolonverbindungen der Formel (11) in guter Reinheit und Ausbeute.

4-Halogenchinazolinverbindungen der Formel (7) und 4-Chinazolonverbindungen der Formel (11) und deren Herstellung werden z.B. in der europäischen Patentveröffentlichung Nr. 33716 beschrieben.

Die Bis-Chinazoline der Formeln (1) bis (5) sind normalerweise farblos oder höchstens schwach gefärbt. Wenn diese Farbbildner mit einem vorzugsweise sauren Entwickler, d.h. einem Elektronenakzeptor, in Kontakt gebracht werden, so ergeben sie intensive gelbe oder orange Farbtöne, die ausgezeichnet lichtecht und vor allem sublimationsecht sind. Sie sind deshalb auch sehr wertvoll im Gemisch mit einem oder mehreren anderen bekannten Farbbildnern, z.B. 3,3-(Bis-amino-phenyl)-phthaliden, 3-Indolyl-3-amino-phenylazaphthaliden, 3,3-(Bis-indolyl)-phthaliden, 3-Aminofluoranen, 2,6-Diaminofluoranen, Leukoauraminen, Spiropyranen, Spirodipyranen, Chromenoindolen, Phenoxazinen, Phenothiazinen, Carbazolylmethanen oder weiteren Triarylmethanleukofarbstoffen, um blaue, graue oder schwarze Färbungen zu ergeben.

Die Bis-Chinazoline der Formeln (1) bis (5) zeigen sowohl auf phenolischen Unterlagen, wie auch besonders auf aktivierten Tonen, eine ausgezeichnete Farbintensität, Sublimations- und Lichtechtheit. Sie eignen sich vor allem als sich schnell entwickelnde Farbbildner für die Verwendung in einem wärmeempfindlichen oder insbesondere druckempfindlichen Aufzeichnungsmaterial, das sowohl Kopier- als auch Registriermaterial sein kann.

Ein druckempfindliches Material besteht beispielsweise aus mindestens einem Paar von Blättern, die mindestens einen Farbbildner der Formeln (1) bis (5), gelöst in einem organischen Lösungsmittel und einen Elektronenakzeptor als Entwickler, enthalten.

Typische Beispiele für solche Entwickler sind Aktivton-Substanzen, wie Attapulgus-Ton, Säureton, Bentonit, Montmorillonit, aktivierter Ton, wie z.B. säureaktiviertes Bentonit oder Montmorillonit, ferner Zeolith, Halloysit, Siliciumdioxyd, Aluminiumoxid, Aluminiumsulfat, Aluminiumphosphat, Zinkchlorid, aktiviertes Kaolin oder irgendein beliebiger Ton. Weitere Entwickler sind sauer reagierende, organische Verbindungen, wie z.B. gegebenenfalls ringsubstituierte Phenole, Salicylsäure oder Salicylsäureester und deren Metallsalze, ferner ein sauer reagierendes, polymeres Material, wie z.B. ein phenolisches Polymerisat, ein Alkylphenolacetylenharz, ein Maleinsäure-Kolophonium-Harz, oder ein teilweise oder vollständig hydrolysiertes Polymerisat von Maleinsäureanhydrid mit Styrol, Ethylen oder Vinylmethylether, oder Carboxypolymethylen. Es können auch Mischungen der genannten polymeren Verbindungen eingesetzt werden. Besonders bevorzugte Entwickler sind Säuretone, Zinksalicylate oder die Kondensationsprodukte von p-substituierten Phenolen mit Formaldehyd. Die letzteren können auch Zink enthalten.

Die Entwickler können zusätzlich auch mit anderen, an sich unreaktiven oder wenig reaktiven Pigmenten oder weiteren Hilfsstoffen wie Kieselgel oder UV-Adsorbern, wie z.B. 2-(2-Hydroxyphenyl)-benzotriazolen gemischt eingesetzt werden. Beispiele für solche Pigmente sind: Talk, Titandioxid, Zinkoxid, Kreide, Tone wie Kaolin sowie organische Pigmente, z.B. Harnstoff-Formaldehyd Kondensate (BET-Oberfläche 2–75 $m^2/g$) oder Melamin-Formaldehyd-Kondensationsprodukte.

Der Farbbildner liefert an den Punkten, an denen er mit dem Elektronenakzeptor in Kontakt kommt, eine gefärbte Markierung. Um zu verhindern, dass die Farbbildner, die in dem druckempfindlichen Aufzeichnungsmaterial enthalten sind, frühzeitig aktiv werden, werden sie in der Regel von dem Elektronenakzeptor getrennt. Dies kann zweckmässig erzielt werden, indem man die Farbbildner in schaum-, schwamm- oder bienenwabenartigen Strukturen einarbeitet. Vorzugsweise sind die Farbbildner in Mikrokapseln eingeschlossen, die sich in der Regel durch Druck zerbrechen lassen.

Wenn die Kapseln durch Druck, beispielsweise mittels eines Bleistiftes, zerbrochen werden und wenn die Farbbildnerlösung auf diese Weise auf ein benachbartes Blatt übertragen wird, das mit einem Elektronenakzeptor beschichtet ist, wird eine farbige Stelle erzeugt. Diese Farbe resultiert aus dem dabei gebildeten Farbstoff, der im sichtbaren Bereich des elektromagnetischen Spektrums absorbiert.

Die Farbbildner werden vorzugsweise in Form von Lösungen in organischen Lösungsmitteln eingekapselt. Beispiele für geeignete Lösungsmittel sind vorzugsweise nichtflüchtige Lösungsmittel, z.B. polyhalogeniertes Paraffin oder Diphenyl, wie Chlorparaffin, Monochlordiphenyl oder Trichlordiphenyl, ferner Tricresylphosphat, Di-n-butylphthalat, Dioctylphthalat, Trichlorbenzol, Trichlorethylphosphat, aromatische Ether, wie Benzylphenylether, Kohlenwasserstofföle, wie Paraffin oder Kerosin, z.B. mit Isopropyl, Isobutyl, sek.-Butyl oder tert.-Butyl alkylierte Derivate von Diphenyl, Naphthalin oder Terphenyl, Dibenzyltoluol, partiell hydriertes Terphenyl, benzylierte Xylole, mono- bis tetramethylierte Diphenylalkane oder weitere chlorierte oder hydrierte, kondensierte, aromatische Kohlenwasserstoffe. Oft werden Mischungen verschiedener Lösungsmittel, insbesondere Mischungen aus Paraffinölen oder Kerosin und Diisopropylnaphthalin oder partiell hydriertem Terphenyl, eingesetzt, um eine optimale Löslichkeit für die Farbbildung, eine rasche und intensive Färbung und eine für die Mikroverkapselung günstige Viskosität zu erreichen.

Die Kapselwände können durch Koazervationskräfte gleichmässig um die Tröpfchen der Farbbildnerlösung herum gebildet werden, wobei das Einkapselungsmaterial z.B. aus Gelatine und Gummi arabicum bestehen kann, wie dies z.B. in der US-Patentschrift 2800457 beschrieben ist. Die Kapseln können vorzugsweise auch aus einem Aminoplast oder modifizierten Aminoplasten durch Polykondensation gebildet werden, wie es in den Britischen Patentschriften 989264, 1156725, 1301052 und 1355124 beschrieben ist. Ebenfalls geeignet sind Mikrokapseln, welche durch Grenzflächenpolymerisation gebildet werden, wie z.B. Kapseln aus Polyester, Polycarbonat, Polysulfonamid, Polysulfonat, besonders aber aus Polyamid oder Polyurethan.

Die Farbbildner der Formeln (1) bis (5) enthaltenden Mikrokapseln können zur Herstellung von druckempfindlichen Kopiermaterialien der verschiedensten bekannten Arten verwendet werden. Die verschiedenen Systeme unterscheiden sich im wesentlichen voneinander durch die Anordnung der Kapseln, der Farbreaktanten und durch das Trägermaterial.

Bevorzugt wird eine Anordnung, bei der der eingekapselte Farbbildner in Form einer Schicht auf der Rückseite eines Übertragungsblattes und der Elektronenakzeptor in Form einer Schicht auf der Vorderseite eines Empfangsblattes vorhanden sind.

Eine andere Anordnung der Bestandteile besteht darin, dass die den Farbbildner enthaltenden Mikrokapseln und der Entwickler in oder auf dem gleichen Blatt in Form einer oder mehrerer Einzelschichten oder in der Papierpulpe vorliegen.

Die Kapseln werden vorzugsweise mittels eines geeigneten Binders auf dem Träger befestigt. Da Papier das bevorzugte Trägermaterial ist, handelt es sich bei diesem Binder hauptsächlich um Papierbeschichtungsmittel, wie Gummi arabicum, Polyvinylalkohol, Hydroxymethylcellulose, Casein, Methylcellulose, Dextrin, Stärke oder Stärkederivate oder Polymerlatices. Letztere sind beispielsweise Butadien-Styrolcopolymerisate oder Acrylhomo- oder copolymere.

Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind, verwendet.

Die Verbindungen der Formeln (1) bis (5) können auch als Farbbildner in einem thermoreaktiven Aufzeichnungsmaterial verwendet werden. Dieses enthält in der Regel mindestens einen Schichtträger, einen Farbbildner, einen Elektronenakzeptor und gegebenenfalls auch ein Bindemittel.

Thermoreaktive Aufzeichnungssysteme umfassen z.B. wärmeempfindliche Aufzeichnungs- und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Messinstrumenten, wie z.B. Elektrocardiographen, verwendet. Die Bilderzeugung (Markierung) kann auch manuell mit einer erhitzten Feder erfolgen. Eine weitere Einrichtung der Erzeugung von Markierungen mittels Wärme sind Laserstrahlen.

Das thermoreaktive Aufzeichnungsmaterial kann so aufgebaut sein, dass der Farbbildner in einer Bindemittelschicht gelöst oder dispergiert ist und in einer zweiten Schicht der Entwickler in dem Bindemittel gelöst und dispergiert ist. Eine andere Möglichkeit besteht darin, dass sowohl der Farbbildner als auch der Entwickler in einer Schicht dispergiert sind. Das Bindemittel wird in spezifischen Bezirken mittels Wärme erweicht und an diesen Punkten, an denen Wärme angewendet wird, kommt der Farbbildner mit dem Elektronenakzeptor in Kontakt und es entwickelt sich sofort die erwünschte Farbe.

Als Entwickler eignen sich die gleichen Elektronenakzeptoren, wie sie in druckempfindlichen Papieren verwendet werden. Beispiele für Entwickler sind die bereits erwähnten Tonminerale und besonders Phenolharze, oder auch phenolische Verbindungen, wie sie beispielsweise in der DE-PS 1251348 beschrieben sind, z.B. 4-tert.-Butylphenol, 4-Phenylphenol, 4-Hydroxydiphenyläther, α-Naphthol, β-Naphthol, 4-Hydroxybenzoesäuremethylester, 4-Hydroxyacetophenon, 2,2'-Dihydroxydiphenyl, 4,4'-Isopropylidendiphenol, 4,4'-Isopropyliden-bis-(2-methylphenol), 2,2-Methylen-bis-(4-phenylphenol), 4,4'-Bis-(hydroxyphenyl)-valeriansäure, Hydrochinon, Pyrogallol, Phloroglucin, p-, m-, o-Hydroxybenzoesäure, Gallussäure, 1-Hydroxy-2-naphthoesäure, sowie Borsäure und organische, vorzugsweise aliphatische Dicarbonsäuren, wie z.B. Weinsäure, Oxalsäure, Maleinsäure, Zitronensäure, Citraconsäure oder Bernsteinsäure.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die Bis-Chinazoline und der Entwickler in Wasser schwer löslich oder unlöslich sind. Das Bindemittel sollte in der Lage sein, den Farbbildner und den Entwickler bei Raumtemperatur zu dispergieren und zu fixieren.

Bei Einwirkung von Wärme erweicht oder schmilzt das Bindemittel, so dass der Farbbildner mit dem Entwickler in Kontakt kommt und sich eine Farbe bilden kann. Wasserlösliche oder mindestens in Wasser quellbare Bindemittel sind z.B. hydrophile Polymerisate, wie Polyvinylalkohol, Polyacrylsäure, Hydroxyethylcellulose, Methylcellulose, Carboxymethylcellulose, Polyacrylamid, Polyvinylpyrrolidon, Gelatine oder Stärke.

Wenn der Farbbildner und der Entwickler in zwei getrennten Schichten vorliegen, können in Wasser unlösliche Bindemittel, d.h. in nichtpolaren oder nur schwach polaren Lösungsmitteln lösliche Bindemittel, wie z.B. Naturkautschuk, synthetischer Kautschuk, chlorierter Kautschuk, Alkydharze, Polystyrol, Styrol/Butadien-Mischpolymerisate, Polymethylacrylate, Ethylcellulose, Nitrocellulose und Polyvinylcarbazol, verwendet werden. Die bevorzugte Anordnung ist jedoch diejenige, bei der der Farbbildner und der Entwickler in einer Schicht in einem wasserlöslichen Bindemittel enthalten sind.

Die thermoreaktiven Schichten können weitere Zusätze enthalten. Zur Verbesserung des Weissgrades, zur Erleichterung des Bedruckens der Papiere und zur Verhinderung des Festklebens der erhitzten Feder können diese Schichten, z.B. Talk, Titandioxyd, Zinkoxyd, Aluminiumhydroxyd, Calciumcarbonat (z.B. Kreide), Tone oder auch organische Pigmente, wie z.B. Harnstoff-Formaldehydpolymerisate, enthalten. Um zu bewirken, dass nur innerhalb eines begrenzten Temperaturbereiches die Farbe gebildet wird, können Substanzen, wie Harnstoff, Thioharnstoff, Diphenylthioharnstoff, Acetamid, Acetanilid, Stearinsäureamid, Phthalsäureanhydrid, Metallstearate, Phthalsäurenitril oder andere entsprechende, schmelzbare Produkte, welche das gleichzeitige Schmelzen des Farbbildners und des Entwicklers induzieren, zugesetzt werden. Bevorzugt enthalten thermographische Aufzeichnungsmaterialien Wachse, z.B. Carnaubawachs, Paraffinwachs, Polyethylenwachs.

In den folgenden Beispielen beziehen sich die angegebenen Prozentsätze, wenn nichts anderes angegeben ist, auf das Gewicht.

Beispiel 1

26,5 g der Chinazolonverbindung der Formel

(i) 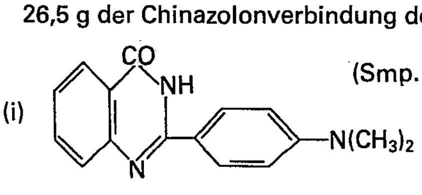 (Smp. 214–217 °C)

werden in 150 g 1,2-Dichlorbenzol bei 150 °C gelöst. Hierauf kühlt man die Lösung auf 90 °C ab und lässt 15,3 g Phosphoroxychlorid im Verlaufe von einer halben Stunde bei 90–95 °C zutropfen. Man rührt die Reaktionsmischung 1 h bei dieser Temperatur und erhält eine dunkelrote Lösung der 4-Chlor-2-(4'-dimethylaminophenyl)-chinazolinverbindung der Formel

(ii) 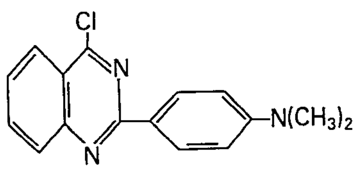

Diese Lösung wird im Verlaufe von einer halben Stunde auf eine Suspension von 11,4 g 4,4'-Isopropylidendiphenol (Bisphenol A) und 2 g Tetrabutylammoniumbromid in 64 g Natriumhydroxidlösung (50%) gegossen. Alsdann kocht man die Reaktionssuspension während einer Stunde unter Rückfluss, worauf das Dichlorbenzol durch Wasserdampfdestillation entfernt wird. Dabei fällt das Produkt kristallin aus, welches bei 50 °C abfiltriert, mit Wasser und Methanol gewaschen und im Vakuum bei 80 °C getrocknet wird. Man erhält 33,1 g einer Bis-Chinazolinverbindung der Formel

(21) 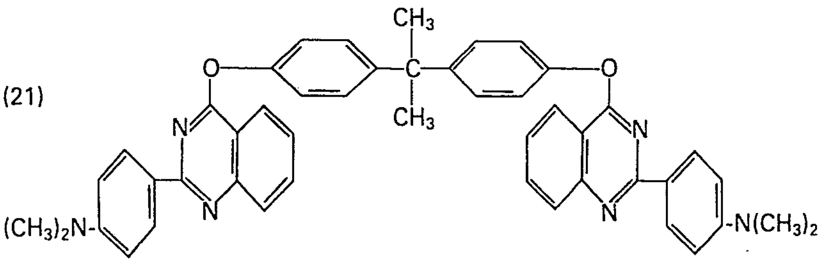

mit einem Schmelzpunkt von 204–206 °C.

Das Remissionsmaximum dieser Bis-Chinazolinverbindung auf Säureton beschichtetes Papier liegt bei 465 nm.

Auf Säuren entwickelt dieser Farbbildner eine gelbe Farbe mit ausgezeichneter Licht- und Sublimierechtheit.

Beispiel 2

Ersetzt man im Beispiel 1 die Chinazolonverbindung der Formel (i) durch 29,3 g der Chinazolonverbindung der Formel

(iii) 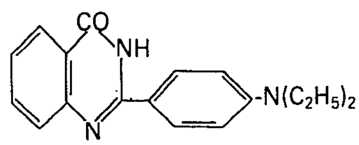

und verfährt im übrigen wie im Beispiel 1 beschrieben, so erhält man 34,2 g einer Bis-Chinazolinverbindung der Formel

(22) 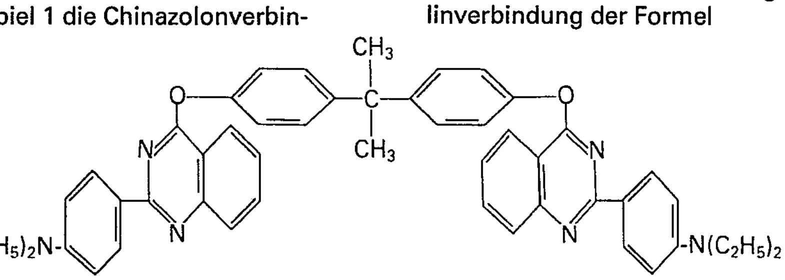

Eine aus Toluol/Methanol umkristallisierte Probe schmilzt bei 147–150 °C. Das Remissionsmaximum auf Säureton beschichtetes Papier liegt bei 470 nm.

Auf Säureton entwickelt dieser Farbbildner eine gelbe Farbe mit ausgezeichneter Licht- und Sublimierechtheit.

Beispiel 3

Ersetzt man im Beispiel 1 die Isopropylidendiphenolverbindung durch 19,2 g einer Bisphenolverbindung der Formel

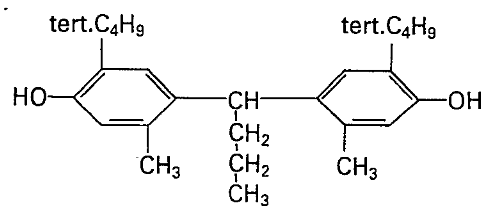

und verfährt im übrigen wie in Beispiel 1 beschrieben, so erhält man 17,2 g einer Bis-Chinazolinverbindung der Formel

(23) 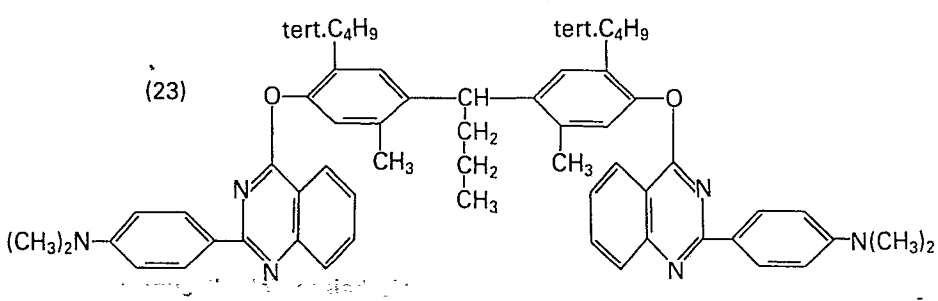

mit einem Schmelzpunkt von 145–160 °C.

Das Remissionsmaximum auf Säureton beschichtetes Papier liegt bei 465 nm.

Auf Säureton entwickelt dieser Farbbildner eine gelbe Farbe mit ausgezeichneter Licht- und Sublimierechtheit.

Auf gleiche Weise wie in den Beispielen 1 bis 3 beschrieben, erhält man unter Verwendung der entsprechenden Ausgangsstoffe die in der folgenden Tabelle aufgeführten Bis-Chinazolinverbindungen der Formel

(24) 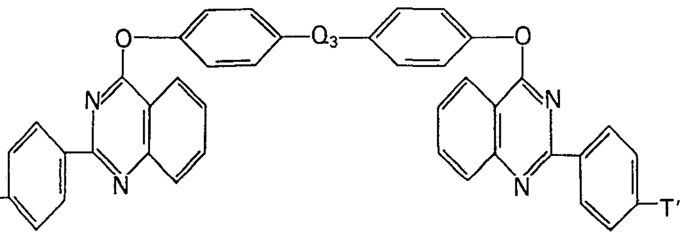

Tabelle

| Beispiel | T' | $Q_3$ | Smp./°C | Farbe auf Säureton |
|---|---|---|---|---|
| | 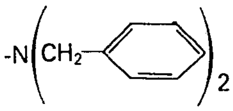 | Isopropyliden | 60–70 | gelb |
| 5 | 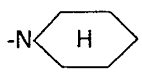 | Isopropyliden | 130–135 | gelb |
| 6 | 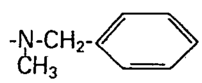 | Isopropyliden | 119–123 | gelb |
| 7 | $-N(n-C_3H_7)_2$ | Isopropyliden | 100–105 | gelb |
| 8 | 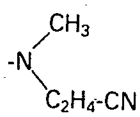 | Isopropyliden | 70–80 | gelb |
| 9 | $-N(CH_3)_2$ | $-CH_2-$ | 209–220 | gelb |
| 10 | $-N(CH_3)_2$ | Cyclohexyliden | 216–225 | gelb |
| 11 | 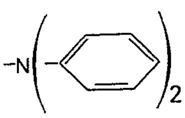 | Isopropyliden | 155–170 | gelb |

Beispiel 12

19 g der Chinazolonverbindung der Formel

(iv) 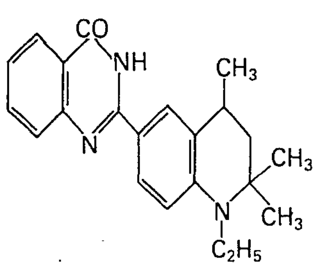

werden bei 150 °C in 90 g 1,2-Dichlorbenzol gelöst. Hierauf lässt man die Lösung auf 95 °C abkühlen und 9 g Phosphoroxychlorid im Verlauf von 30 min bei dieser Temperatur zutropfen. Alsdann wird die Reaktionsmischung noch 1 h bei 90–95 °C gerührt. Die entstandene rote Lösung der 4-Chlor-2-tetrahydrochinolinyl-chinazolinverbindung der Formel

(v) 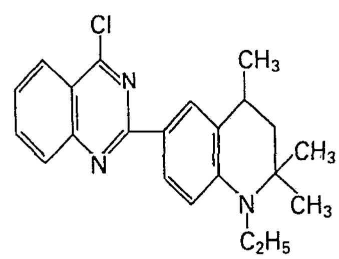

wird im Verlauf von 15 min auf eine Suspension, bestehend aus 11,4 g 4,4'-Isopropylidendiphenol, 2,2 g Tetrabutylammoniumbromid und 35 g einer 50%igen wässrigen Natriumhydroxidlösung gegossen, worauf die Temperatur auf 110 °C ansteigt. Man rührt die Reaktionsmischung noch 1 h bei 100–110 °C und entfernt das 1,2-Dichlorbenzol durch Wasserdampfdestillation. Das ausgefallene Produkt wird unter Erwärmen in Toluol gelöst und die heisse Toluollösung über Aktivkohle filtriert. Beim Abkühlen kristallisiert das Produkt aus und wird abfiltriert und getrocknet. Man erhält 12 g einer Verbindung der Formel

(25)

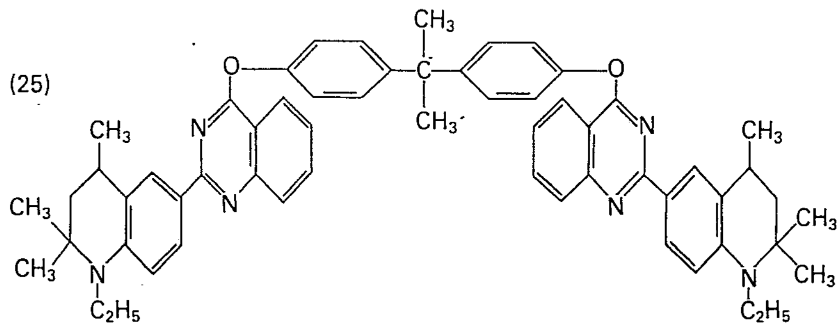

mit einem Schmelzpunkt von 193–196 °C.

Auf Säureton entwickelt dieser Farbbildner eine goldgelbe Farbe mit guter Licht- und Sublimierechtheit.

Die im Beispiel 12 verwendete Chinazolonverbindung der Formel (iv) kann wie folgt hergestellt werden:

23,1 g N-Ethyl-2,2,4-trimethyl-tetrahydrochinolin-6-aldehyd werden in 150 ml Ethanol gelöst. Man gibt zur Lösung 13,6 g Anthranilsäureamid und 4 ml Schwefelsäure (10%) zu und erwärmt die Reaktionsmischung auf 60 °C. Man hält die Mischung 1 h bei 60 °C und oxidiert das Produkt durch Zutropfen von 69 g einer 40%igen wässrigen Natriumbisulfitlösung und anschliessendes Rühren während 2 h bei Rückflusstemperatur. Nach Abkühlung auf Raumtemperatur wird das ausgefallene Produkt abfiltriert, mit Ethanol gewaschen und getrocknet. Man erhält 19 g der Chinazolonverbindung der Formel (iv) mit einem Schmelzpunkt von 215–219 °C.

Beispiel 13: Herstellung eines druckempfindlichen Kopierpapiers

Eine Lösung von 3 g der Bis-Chinazolinverbindung der Formel (21) in 80 g partiell hydriertem Terphenyl und 17 g Kerosin wird auf an sich bekannte Weise mit Gelatine und Gummi arabicum durch Koazervation mikroverkapselt, mit Stärkelösung vermischt und auf ein Blatt Papier gestrichen. Ein zweites Blatt Papier wird auf der Frontseite mit säureaktiviertem Bentonit als Farbentwickler beschichtet. Das erste Blatt und das mit Farbentwickler beschichtete Papier werden mit den Beschichtungen benachbart aufeinandergelegt. Durch Schreiben mit der Hand oder mit der Schreibmaschine auf dem ersten Blatt wird Druck ausgeübt, und es entwickelt sich sofort auf dem mit dem Entwickler beschichteten Blatt eine intensive gelbe Kopie, die ausgezeichnet sublimier- und lichtecht ist.

Entsprechende intensive, sublimier- und lichtechte gelbe Kopien werden auch bei Verwendung jedes der anderen in den Herstellungsbeispielen angegebenen Farbbildner der Formeln (22) bis (25) erzielt.

Beispiel 14

Ersetzt man in Beispiel 13 die Bis-Chinazolinverbindung der Formel (21) durch eine Mischung der folgenden Zusammensetzung: 1,2 g 3,3-Bis-(4'-dimethylaminophenyl)-6-dimethylaminophthalid, 1,2 g N-Butylcarbazol-3-yl-bis-(4'-N-methyl-N-phenylaminophenyl)-methan, 1,5 g der Bis-Chinazolinverbindung der Formel (21) und 0,4 g 3,3-Bis-(N-n-octyl-2'-methylindol-3'-yl)-phthalid und verfährt im übrigen wie in Beispiel 13 beschrieben, so erhält man ein druckempfindliches Aufzeichnungsmaterial, welches durch Schreiben mit der Hand oder mit der Schreibmaschine eine intensive und lichtechte schwarze Kopie ergibt.

Beispiel 15

1 g der Bis-Chinazolinverbindung der Formel (22) wird in 17 g Toluol gelöst. Zu dieser Lösung gibt man unter Rühren 12 g Polyvinylacetat, 8 g Calciumcarbonat und 2 g Titandioxyd. Die erhaltene Suspension wird im Gewichtsverhältnis 1:1 mit Toluol verdünnt und mit einem 10 µm Rakel auf ein Blatt Papier gestrichen. Auf dieses Blatt Papier wird ein zweites Blatt Papier gelegt, dessen Unterseite bei einem Auftragsgewicht von 3 $g/m^2$ mit einer Mischung, bestehend aus 1 Teil eines Amidwachses, 1 Teil eines Stearinwachses und 1 Teil Zinkchlorid beschichtet worden ist. Durch Schreiben mit der Hand oder mit der Schreibmaschine auf dem oberen Blatt wird Druck ausgeübt, und es entwickelt sich sofort auf dem mit dem Farbbildner beschichteten Blatt eine intensive, sublimier- und lichtechte gelbe Farbe.

Beispiel 16: Herstellung eines wärmeempfindlichen Aufzeichnungsmaterials

In einer Kugelmühle werden 32 g 4,4'-Isopropylidendiphenol (Bisphenol A), 3,8 g Distearylamid des Ethylendiamins, 39 g Kaolin, 20 g eines zu 88% hydrolysierten Polyvinylalkohols und 500 ml Wasser gemahlen, bis die Teilchengrösse ca. 5 µm beträgt. In einer zweiten Kugelmühle werden 6 g der Verbindung der Formel (21), 3 g eines zu 88% hydrolysierten Polyvinylalkohols und 60 ml Wasser zu einer Teilchengrösse von ca. 3 µm gemahlen.

Die beiden Dispersionen werden zusammengegeben und mit einem Trockenauftragsgewicht von 5,5 $g/m^2$ auf ein Papier gestrichen. Durch Berührung des Papiers mit einem erhitzten Kugelschreiber wird eine intensive gelbe Farbe erhalten, die eine ausgezeichnete Sublimier- und Lichtechtheit hat.

Intensive und lichtechte gelbe Farben können auch bei Verwendung jeder der anderen Farbbildner der Formeln (22) bis (25) erhalten werden.

Beispiel 17

In einer Kugelmühle werden 2,7 g der Bis-Chinazolinverbindung der Formel (22), 24 g N-Phenyl-N'-(1-hydroxy-2,2,2-trichloroethyl)-harnstoff, 16 g

Stearinsäureamid, 59 g eines zu 88% hydrolysierten Polyvinylalkohols und 58 ml Wasser gemahlen, bis die Teilchengrösse 2–5 μm beträgt. Diese Suspension wird bei einem Trockenauftragsgewicht von 5,5 g/m² auf ein Blatt Papier gestrichen. Durch Berührung des Papiers mit einem erhitzten Kugelschreiber wird eine intensive, sublimier- und lichtechte gelbe Farbe erhalten.

**Patentansprüche**

1. Chromogene Bis-Chinazolinverbindungen der Formel

(1)

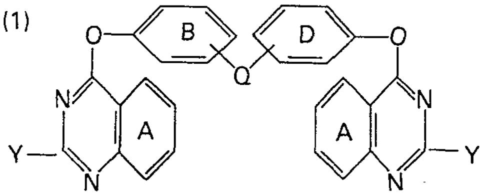

worin

die Ringe A, B und D, unabhängig voneinander, unsubstituiert oder durch Cyano, Nitro, Halogen, Niederalkyl, Phenyl, Benzyl, Niederalkoxy oder Niederalkoxycarbonyl substituiert sind,

Q die direkte Bindung, einen aliphatischen oder cycloaliphatischen Kohlenwasserstoffrest mit höchstens 8 Kohlenstoffatomen, -CO-, -S- oder $-SO_2-$ und

Y den Rest der Formel

(2a)

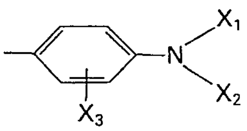

oder

den Rest der Formel

(2b)

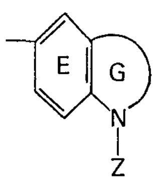

bedeuten, worin

$X_1$ und $X_2$, unabhängig voneinander, je Wasserstoff, unsubstituiertes oder durch Halogen, Hydroxy, Cyano oder Niederalkoxy substituiertes Alkyl mit höchstens 12 Kohlenstoffatomen, Cycloalkyl, Phenyl, Benzyl oder durch Halogen, Nitro, Cyano, Niederalkyl, Niederalkoxy oder Niederalkoxycarbonyl substituiertes Phenyl oder Benzyl

oder $X_1$ und $X_2$ zusammen mit dem sie verbindenden Stickstoffatom einen fünf- oder sechsgliedrigen heterocyclischen Rest,

$X_3$ Wasserstoff, Halogen, Nitro, Niederalkyl, Niederalkoxy oder Niederalkoxycarbonyl und

Z Wasserstoff oder unsubstituiertes oder durch Halogen, Cyano oder Niederalkoxy substituiertes Alkyl mit höchstens 8 Kohlenstoffatomen, Cycloalkyl oder Benzyl bedeuten

und worin der Ring E unsubstituiert oder durch Cyano, Halogen, Niederalkyl oder Niederalkoxy substituiert ist und der Ring G einen hydrierten fünf- oder sechsgliedrigen Stickstoffheterocyclus darstellt, der gegebenenfalls als Ringglied ein weiteres Heteroatom aufweist und unsubstituiert oder durch Halogen, Cyano, Hydroxyl, Niederalkyl, Niederalkoxy, $C_5$–$C_6$-Cycloalkyl, Benzyl oder $C_3$–$C_6$-Alkylen einfach oder je nach Substituenten mehrfach C-substituiert ist, wobei die Bezeichnung Nieder $C_1$–$C_5$- darstellt.

2. Bis-Chinazolinverbindungen gemäss Anspruch 1, dadurch gekennzeichnet, dass in Formel (1) die Ringe A, B und D unsubstituiert sind.

3. Bis-Chinazolinverbindungen gemäss einem der Ansprüche 1 und 2, dadurch gekennzeichnet, dass in Formel (1) Y den Rest der Formel (2a) bedeutet.

4. Bis-Chinazolinverbindungen gemäss Anspruch 3, dadurch gekennzeichnet, dass in Formel (2a) $X_1$ und $X_2$, unabhängig voneinander, je Niederalkyl, Benzyl, Phenyl, Niederalkylphenyl oder Niederalkoxyphenyl bedeuten.

5. Bis-Chinazolinverbindungen gemäss einem der Ansprüche 1 und 2, dadurch gekennzeichnet, dass in Formel (1) Y den Rest der Formel (2b) bedeutet, worin der Ring G sechsgliedrig ist.

6. Bis-Chinazolinverbindungen gemäss einem der Ansprüche 1 und 2, dadurch gekennzeichnet, dass in Formel (1) Y den Rest der Formel (2b) bedeutet, worin Z Niederalkyl, Benzyl oder β-Cyanoethyl, bedeutet.

7. Bis-Chinazolinverbindungen gemäss einem der Ansprüche 1 bis 6, dadurch gekennzeichnet, dass in Formel (1) Q den aliphatischen oder cycloaliphatischen Kohlenwasserstoffrest bedeutet.

8. Bis-Chinazolinverbindungen gemäss Anspruch 1, dadurch gekennzeichnet, dass sie der Formel

(3)

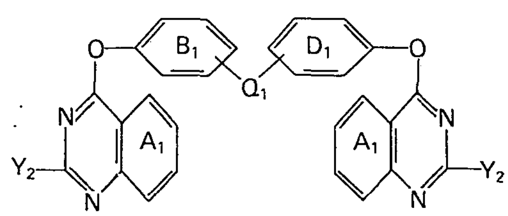

entsprechen, worin

$Q_1$ die direkte Bindung, einen geradkettigen oder verzweigten Alkylen- oder Alkylidenrest je mit höchstens 8, vorzugsweise höchstens 4 Kohlenstoffatomen oder einen Cyclohexylidenrest

$Y_2$ einen Aminophenylrest der Formel

(3a)

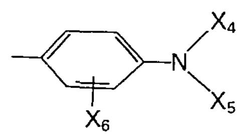

einen 5-Indolinrest der Formel

(3b)

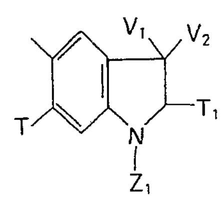

einen Tetrahydrochinolinylrest der Formel

(3c)

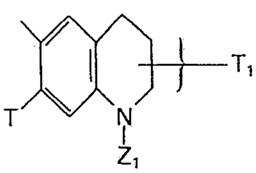

einen Tetrahydrochinolinylrest der Formel

(3d)

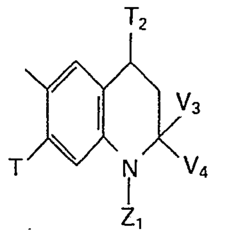

oder einen Benzomorpholinorest der Formel

(3e)

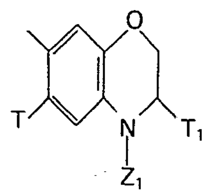

bedeuten, wobei

$X_4$ und $X_5$, unabhängig voneinander, Niederalkyl, Cyano-Niederalkyl, Benzyl, Phenyl, Niederalkylphenyl oder Niederalkoxyphenyl oder

$X_4$ und $X_5$ zusammen mit dem sie verbindenden Stickstoffatom, Pyrrolidino, Piperidino oder Morpholino,

$X_6$ Wasserstoff, Halogen, Niederalkyl oder Niederalkoxy,

$Z_1$ Wasserstoff, $C_1$–$C_8$-Alkyl, $C_2$–$C_6$-Alkoxyalkyl, β-Cyanoethyl oder Benzyl,

T Wasserstoff, Halogen, Niederalkyl oder Niederalkoxy,

$T_1$ und $T_2$ je Wasserstoff, Halogen, Hydroxy, Niederalkyl oder Niederalkoxy und

$V_1$, $V_2$, $V_3$ und $V_4$ je Wasserstoff, Niederalkyl, $C_5$–$C_6$-Cycloalkyl oder Benzyl oder ($V_1$ und $V_2$) oder ($V_3$ und $V_4$) je zusammen $C_3$–$C_6$-Alkylen bedeuten und worin

die Ringe $A_1$, $B_1$ und $D_1$, unabhängig voneinander, unsubstituiert oder durch einen oder zwei Substituenten ausgewählt aus Cyano, Halogen, Niederalkyl, Phenyl und Niederalkoxy substituiert sind.

9. Bis-Chinazolinverbindungen gemäss Anspruch 8, dadurch gekennzeichnet, dass in Formel (3) $Y_2$ einen Rest der Formel (3a) bedeutet.

10. Bis-Chinazolinverbindungen gemäss Anspruch 9, dadurch gekennzeichnet, dass in Formel (3a) $X_4$ und $X_5$ Niederalkyl oder Benzyl, $X_6$ Wasserstoff bedeutet.

11. Bis-Chinazolinverbindungen gemäss einem der Ansprüche 8 bis 10, dadurch gekennzeichnet, dass in Formel (3) $Q_1$ Alkylen oder Alkyliden je mit höchstens 4 Kohlenstoffatomen, insbesondere Isopropyliden oder Butyliden bedeutet.

12. Bis-Chinazolinverbindungen gemäss einem der Ansprüche 8 bis 11, dadurch gekennzeichnet, dass in Formel (3) der Ring $A_1$ unsubstituiert ist und die Ringe $B_1$ und $D_1$ unsubstituiert oder durch Methyl und/oder tert.-Butyl substituiert sind.

13. Bis-Chinazolinverbindungen gemäss Anspruch 8, dadurch gekennzeichnet, dass in Formel (3) $Y_2$ den Rest der Formel (3d) bedeutet.

14. Bis-Chinazolinverbindungen gemäss Anspruch 1 oder 8, dadurch gekennzeichnet, dass sich in Formeln (1) bzw. (3) Q und $Q_1$ in para-Stellung zu den O-Atomen befinden.

15. Bis-Chinazolinverbindungen gemäss Anspruch 1 oder 8, dadurch gekennzeichnet, dass sie der Formel

(5)

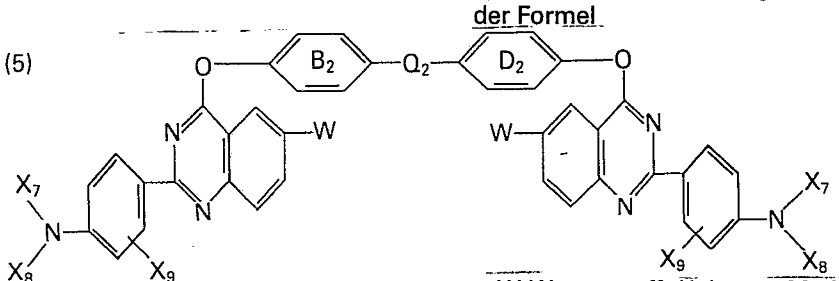

entsprechen, worin

$Q_2$ geradkettiges oder verzweigtes Alkylen oder Alkyliden jeweils mit höchstens 4 Kohlenstoffatomen, oder Cyclohexyliden,

$X_7$ und $X_8$ Niederalkyl, β-Cyanoethyl, Benzyl oder Phenyl oder -$NX_7X_8$ Piperidino,

$X_9$ Wasserstoff, Methyl, Methoxy oder Ethoxy und

W Wasserstoff, Halogen, Methyl oder Methoxy bedeuten und die Ringe $B_2$ und $D_2$ unsubstituiert oder durch einen oder zwei Substituenten ausgewählt aus Methyl, Methoxy und tert.-Butyl substituiert sind.

16. Bis-Chinazolinverbindungen gemäss Anspruch 1 oder 8, dadurch gekennzeichnet, dass sie der Formel

(4)

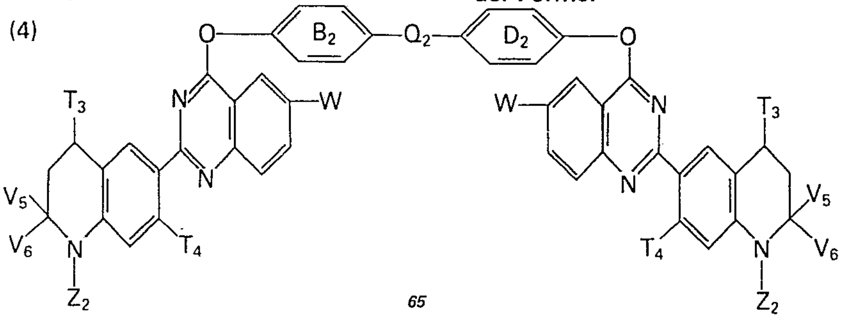

entsprechen, worin

$Q_2$ geradkettiges oder verzweigtes Alkylen oder Alkyliden je mit höchstens 4 Kohlenstoffatomen,

$Z_2$ Alkyl mit 1 bis 8 Kohlenstoffatomen, β-Cyanoethyl oder Benzyl,

$T_3$, $V_5$ und $V_6$ je Niederalkyl,

$T_4$ Wasserstoff oder Methyl und

W Wasserstoff, Halogen, Methyl oder Methoxy bedeuten und die Ringe

$B_2$ und $D_2$ unsubstituiert oder durch einen oder zwei Substituenten ausgewählt aus Methyl, Methoxy oder tert.-Butyl substituiert sind.

17. Verfahren zur Herstellung von Bis-Chinazolinverbindungen der in Anspruch 1 angegebenen Formel (1), dadurch gekennzeichnet, dass man 1 Mol einer Bis-Phenolverbindung der Formel

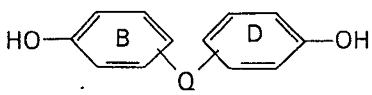

worin B, D und Q die im Anspruch 1 angegebene Bedeutung haben, mit 2 Mol einer 4-Halogenchinazolinverbindung der Formel

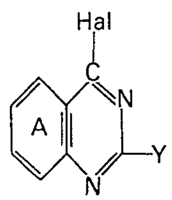

worin A und Y die in Anspruch 1 angegebene Bedeutung haben und Hal Halogen bedeutet, umsetzt.

18. Druck- oder wärmeempfindliches Aufzeichnungsmaterial, dadurch gekennzeichnet, dass es in seinem Farbreaktantensystem als Farbbildner mindestens eine Bis-Chinazolinverbindung der in einem der Ansprüche 1 bis 16 angegebenen Formel enthält.

19. Druckempfindliches Aufzeichnungsmaterial gemäss Anspruch 18, dadurch gekennzeichnet, dass es die Bis-Chinazolinverbindung, gelöst in einem organischen Lösungsmittel, und mindestens einen festen Elektronenakzeptor enthält.

20. Druckempfindliches Aufzeichnungsmaterial gemäss einem der Ansprüche 18 und 19, dadurch gekennzeichnet, dass die Bis-Chinazolinverbindung in Mikrokapseln eingekapselt ist.

21. Druckempfindliches Aufzeichnungsmaterial gemäss Anspruch 20, dadurch gekennzeichnet, dass die eingekapselte Bis-Chinazolinverbindung in Form einer Schicht auf der Rückseite eines Übertragungsblattes und der Elektronenakzeptor in Form einer Schicht auf der Vorderseite des Empfangsblattes vorhanden sind.

22. Druckempfindliches Aufzeichnungsmaterial gemäss einem der Ansprüche 18 bis 21, dadurch gekennzeichnet, dass die Bis-Chinazolinverbindung gemeinsam mit einem oder mehreren anderen Farbbildnern enthalten ist.

23. Wärmeempfindliches Aufzeichnungsmaterial gemäss Anspruch 18, dadurch gekennzeichnet, dass es in mindestens einer Schicht mindestens einen Farbbildner, einen Elektronenakzeptor und gegebenenfalls ein Bindemittel enthält, worin der Farbbildner die in einem der Ansprüche 1 bis 16 angegebene Formel hat.

**Claims**

1. A chromogenic bisquinazoline of the formula

(1)

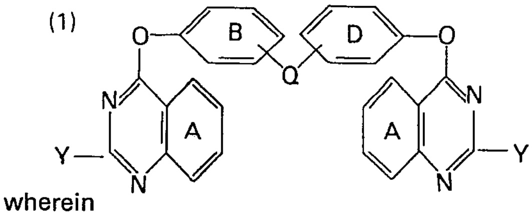

wherein

the rings A, B and D each independently are unsubstituted or substituted by cyano, nitro, halogen, lower, alkyl, phenyl, benzyl, lower alkoxy or lower alkoxycarbonyl

Q is the direct bond, an aliphatic or cycloaliphatic hydrocarbon radical containing not more than 8 carbon atoms, or is -CO-, -S- or $-SO_2-$ and

Y is the radical of the formula

(2a)

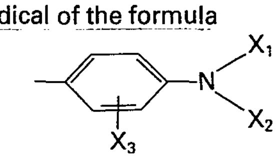

or the radical of the formula

(2b)

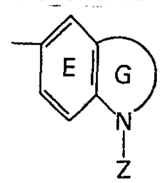

wherein

$X_1$ and $X_2$ are each independently hydrogen, alkyl containing not more than 12 carbon atoms which is unsubstituted or substituted by halogen, hydroxyl, cyano or lower alkoxy, or are cycloalkyl, phenyl, benzyl, or phenyl or benzyl each substituted by halogen, nitro, cyano, lower alkyl, lower alkoxy or lower alkoxycarbonyl or

$X_1$ and $X_2$, together with the nitrogen atom to which they are attached, are a 5- or 6-membered heterocyclic radical,

$X_3$ is hydrogen, halogen, nitro, lower alkyl, lower alkoxy or lower alkoxycarbonyl, and

Z is hydrogen or alkyl containing not more than 8 carbon atoms which is unsubstituted or substituted by halogen, cyano or lower alkoxy, or is cycloalkyl or benzyl,

and the ring E is unsubstituted or substituted by cyano, halogen, lower alkyl or lower alkoxy, and the ring G is a hydrogenated 5- or 6-membered N-heterocyclic ring system which may contain a further hetero atom as ring member and which is unsubstituted or C-substituted by one or, depending on the substituents, by more than one of the same or different substituents selected from the group consisting of halogen, cyano, hydroxyl, lower alkyl, lower alkoxy, $C_5$–$C_6$-cycloalkyl, benzyl or $C_3$–$C_6$-alkylene, the term «lower» signifying $C_1$–$C_5$.

2. A bisquinazoline according to claim 1, wherein the rings A, B and D in formula (1) are unsubstituted.

3. A bisquinazoline according to either of claims 1 or 2, wherein Y in formula (1) is the radical of the formula (2a).

4. A bisquinazoline according to claim 3, wherein $X_1$ and $X_2$ in formula (2a) are each independently lower alkyl, benzyl, phenyl, lower alkylphenyl or lower alkoxyphenyl.

5. A bisquinazoline according to either of claims 1 or 2, wherein Y in formula (1) is the radical of the formula (2b), wherein the ring G is 6-membered.

6. A bisquinazoline according to either of claims 1 or 2, wherein Y in formula (1) is the radical of the formula (2b), wherein Z is lower alkyl, benzyl or β-cyanoethyl.

7. A bisquinazoline according to any one of claims 1 to 6, wherein Q in formula (1) is the aliphatic or cycloaliphatic hydrocarbon radical.

8. A bisquinazoline according to claim 1, of the formula

(3) 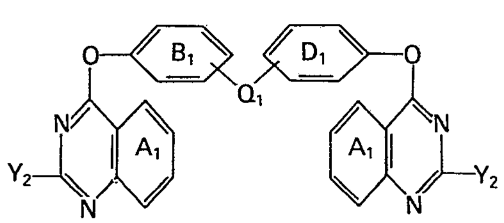

wherein

$Q_1$ is the direct bond, a straight chain or branched alkylene or alkylidene radical, each containing not more than 8 carbon atoms, preferably not more than 4 carbon atoms, or is a cyclohexylidene radical,

$Y_2$ is an aminophenyl radical of the formula

(3a) 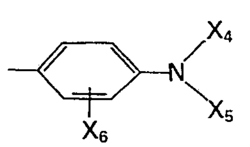

a 5-indoline radical of the formula

(3b) 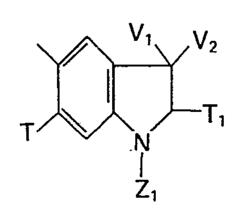

a tetrahydroquinolinyl radical of the formula

(3c) 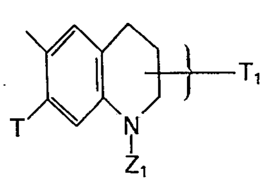

a tetrahydroquinolinyl radical of the formula

(3d) 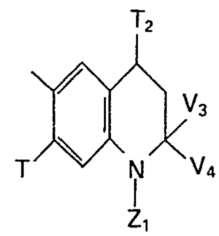

or a benzomorpholino radical of the formula

(3e) 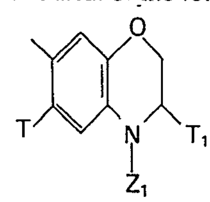

in which formulae

$X_4$ and $X_5$ are each independently lower alkyl, cyano-lower alkyl, benzyl, phenyl, lower alkylphenyl or lower alkoxyphenyl, or

$X_4$ and $X_5$, together with the nitrogen atom to which they are attached, are pyrrolidino, piperidino or morpholino,

$X_6$ is hydrogen, halogen, lower alkyl or lower alkoxy,

$Z_1$ is hydrogen, $C_1$–$C_8$-alkyl, $C_2$–$C_6$-alkoxyalkyl, β-cyanoethyl or benzyl,

T is hydrogen, halogen, lower alkyl or lower alkoxy,

$T_1$ and $T_2$ are each hydrogen, halogen, hydroxy, lower alkyl or lower alkoxy, and

$V_1$, $V_2$, $V_3$ and $V_4$ are each hydrogen, lower alkyl, $C_5$–$C_6$-cycloalkyl or benzyl, or ($V_1$ and $V_2$) or ($V_3$ and $V_4$) are each together $C_3$–$C_6$-alkylene,

and the rings $A_1$, $B_1$ and $D_1$ are each independently unsubstituted or substituted by one or two members selected from the group consisting of cyano, halogen, lower alkyl, phenyl and lower alkoxy.

9. A bisquinazoline according to claim 8, wherein $Y_2$ in formula (3) is a radical of the formula (3a).

10. A bisquinazoline according to claim 9, wherein $X_4$ and $X_5$ in formula (3a) are each lower alkyl or benzyl and $X_6$ in said formula is hydrogen.

11. A bisquinazoline according to any one of claims 8 to 10, wherein $Q_1$ in formula (3) is alkylene or alkylidene, each containing not more than 4 carbon atoms, in particular isopropylidene or butylidene.

12. A bisquinazoline according to any one of claims 8 to 11, wherein the ring $A_1$ in formula (3) is unsubstituted and the rings $B_1$ and $D_1$ in said formula are unsubstituted or substituted by one or two substituents selected from methyl and tert-butyl.

13. A bisquinazoline according to claim 8, wherein $Y_2$ in formula (3) is the radical of the formula (3d).

14. A bisquinazoline according to either of claims 1 or 8, wherein Q in formula (1) or (3) is in the para-position with respect to the oxygen atoms.

15. A bisquinazoline according to either of claims 1 or 8, of the formula

(5)

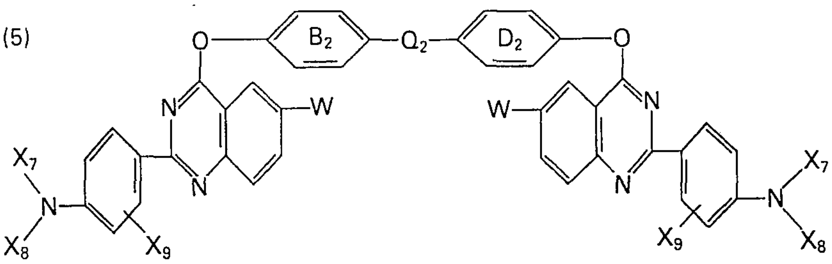

wherein

$Q_2$ is straight chain or branched alkylene or alkylidene, each containing not more than 4 carbon atoms, or is cyclohexylidene,

$X_7$ and $X_8$ are each lower alkyl, β-cyanoethyl, benzyl or phenyl, or $-NX_7X_8$ is piperidino,

$X_9$ is hydrogen, methyl, methoxy or ethoxy

W is hydrogen, halogen, methyl or methoxy and the rings $B_2$ and $D_2$ are unsubstituted or substituted by one or two substituents selected from the group consisting of methyl, methoxy and tert-butyl.

16. A bisquinazoline according to either of claims 1 or 8, of the formula

(4)

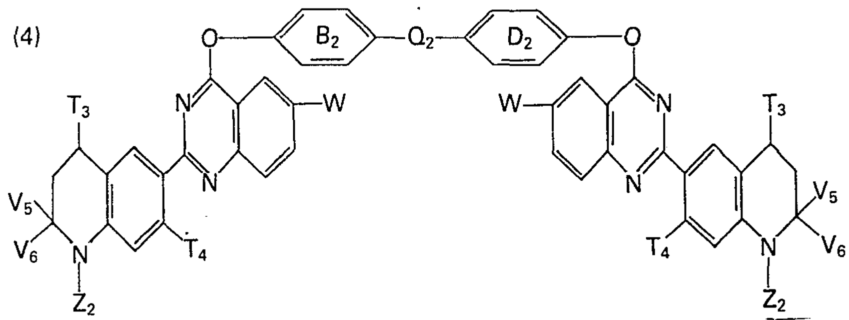

wherein

$Q_2$ is straight chain or branched alkylene or alkylidene, each containing not more than 4 carbon atoms,

$Z_2$ is $C_1$–$C_8$-alkyl, β-cyanoethyl or benzyl,

$T_3$, $V_5$ and $V_6$ are each lower alkyl,

$T_4$ is hydrogen or methyl, and

W is hydrogen, halogen, methyl or methoxy, and the rings $B_2$ and $D_2$ are unsubstituted or substituted by one or two substituents selected from the group consisting of methyl, methoxy and tert-butyl.

17. A process for the preparation of a bisquinazoline of the formula (1) as indicated in claim 1, which process comprises reacting 1 mole of a bisphenol of the formula

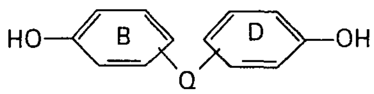

wherein B, D and Q are as defined in claim 1, with 2 moles of a 4-haloquinazoline of the formula

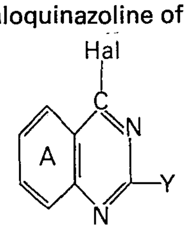

wherein A and Y are as defined in claim 1 and Hal is halogen.

18. A pressure-sensitive or heat-sensitive recording material which comprises a support which contains, or has coated thereon as colour former, at least one bisquinazoline of the formula as indicated in any one of claims 1 to 16.

19. The pressure-sensitive recording material of claim 18, wherein the bisquinazoline is dissolved in an organic solvent, and which recording material further comprises at least one solid electron acceptor.

20. The pressure-sensitive recording material of either of claims 18 or 19, wherein the bisquinazoline is encapsulated in microcapsules.

21. The pressure-sensitive recording material of claim 20, wherein the encapsulated bisquinazoline is present in the form of a layer on the back of a transfer sheet and the electron acceptor is present in the form of a layer on the face of a receiver sheet.

22. The pressure-sensitive recording material of any one of claims 18 to 21, which comprises the bisquinazoline together with one or more other colour formers.

23. The heat-sensitive recording material of claim 18, which comprises in at least one layer, at least one colour former of the formula as indicated in any one of claims 1 to 16, at least one electron acceptor and at least one binder.

**Revendications**

1. Composés bis-quinazoliniques chromogènes, de formule

(1)

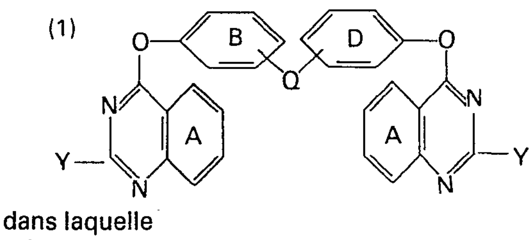

dans laquelle

les cycles A, B et D, indépendamment les uns des autres, sont chacun non-substitués ou sont

substitués par des groupes cyano, nitro, halogène, alkyle, inférieur, phényle, benzyle, alcoxy inférieur ou alcoxy-carbonyle inférieur,

Q représente la liaison directe, un radical hydrocarboné aliphatique ou cycloaliphatique contenant au plus 8 atomes de carbone, -CO-, -S- ou $-SO_2-$, et

Y est le radical de formule

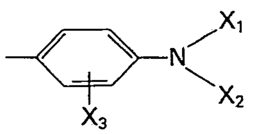

ou bien le radical de formule

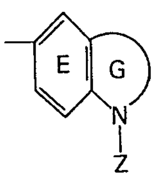

dans lesquelles

$X_1$ et $X_2$, indépendamment l'un de l'autre, sont chacun l'hydrogène; un radical alkyle ayant au plus 12 atomes de carbone, non substitué ou substitué par un groupe halogène, hydroxy, cyano ou alcoxy inférieur; un radical cycloalkyle; le radical phényle; le radical benzyle; ou un radical phényle ou benzyle substitué par un halogène, un groupe nitro, cyano, alkyle inférieur, alcoxy inférieur ou alcoxycarbonyle inférieur,

ou bien $X_1$ et $X_2$, avec l'atome d'azote qui les relie, forment un radical hétérocyclique à 5 ou 6 chaînons,

$X_3$ est l'hydrogène, un halogène, un groupe nitro, alkyle inférieur, alcoxy inférieur ou alcoxycarbonyle inférieur, et

Z est l'hydrogène ou un radical alkyle ayant au plus 8 atomes de carbone, non-substitué ou substitué par un halogène, un groupe cyano ou alcoxy inférieur; un radical cycloalkyle ou benzyle; et où le cycle G représente un hétérocycle azoté, hydrogéné, à 5 ou 6 chaînons, lequel présente éventuellement en tant que chaînon du cycle un autre hétéroatome, et est non-substitué, ou une fois ou encore, selon les substituants, plusieurs fois substitué sur des atomes de carbone par un halogène, un groupe cyano, hydroxyle, alkyle inférieur, alcoxy inférieur, cycloalkyle en $C_5$–$C_6$, benzyle ou alkylène en $C_3$–$C_6$, la désignation «inférieur» correspondant à $C_1$–$C_5$.

2. Dérivés bis-quinazoliniques selon la revendication 1, caractérisés en ce que, dans la formule (1), les cycles A, B et D sont non-substitués.

3. Dérivés bis-quinazoliniques selon l'une des revendications 1 et 2, caractérisés en ce que, dans la formule (1), Y représente le radical de formule (2a).

4. Composés bis-quinazoliniques selon la revendication 3, caractérisés en ce que, dans la formule (2a), $X_1$ et $X_2$, indépendamment l'un de l'autre, désignent chacun un radical alkyle inférieur, benzyle, phényle, (alkyle inférieur)-phényle ou (alcoxy inférieur)-phényle.

5. Composés bis-quinazoliniques selon l'une des revendications 1 et 2, caractérisés en ce que, dans la formule (1), Y désigne le radical de formule (2b) dans laquelle le cycle G est à six chaînons.

6. Composés bis-quinazoliniques selon l'une des revendications 1 et 2, caractérisés en ce que, dans la formule (1), Y représente le radical de formule (2b) dans laquelle Z est un radical alkyle inférieur, benzyle ou bêta-cyanoéthyle.

7. Composés bis-quinazoliniques selon l'une des revendications 1 à 6, caractérisés en ce que, dans la formule (1), Q désigne un radical hydrocarboné aliphatique ou aromatique.

8. Composés bis-quinazoliniques selon la revendication 1, caractérisés en ce qu'ils correspondent à la formule

(3) 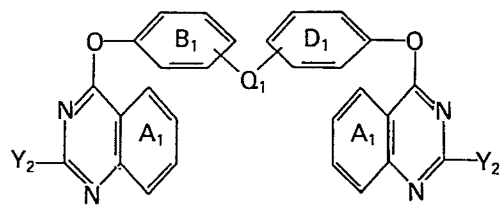

dans laquelle

Q est la liaison directe, un radical alkylène ou alkylidène, à chaîne droite ou ramifiée, ayant chacun au plus 8, de préférence au plus 4 atomes de carbone, ou un radical cyclohexylidène,

$Y_2$ est un radical aminophényle de formule

(3a) 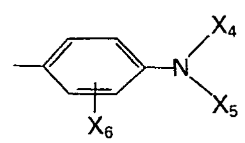

un radical 5-indolinyle de formule

(3b) 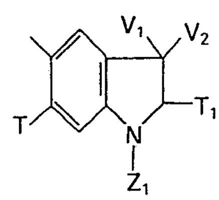

un radical tétrahydroquinoléinyle de formule

(3c) 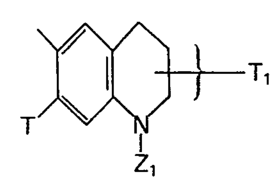

un radical tétrahydroquinoléinyle de formule

(3d) 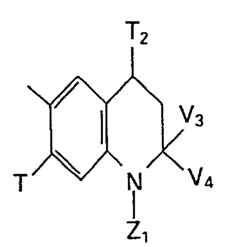

ou un radical benzomorpholinyle de formule

(3e)

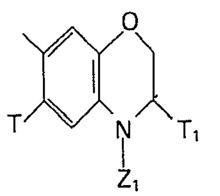

dans lesquelles

$X_4$ et $X_5$, indépendamment l'un de l'autre, sont chacun un radical alkyle inférieur, cyano-alkyle inférieur, benzyle, phényle (alkyle inférieur)-phényle ou (alcoxy inférieur)-phényle ou bien

$X_4$ et $X_5$ forment, avec l'atome d'azote qui les relie, un cycle pyrrolidino, pipéridino ou morpholino,

$X_6$ est l'hydrogène, un halogène, un radical alkyle inférieur ou alcoxy inférieur,

$Z_1$ est l'hydrogène, un radical alkyle en $C_1$–$C_8$, alcoxyalkyle en $C_2$–$C_6$, le radical β-cyanoéthyle ou benzyle,

T est l'hydrogène, un halogène, un radical alkyle inférieur ou alcoxy inférieur,

$T_1$ et $T_2$ sont chacun l'hydrogène, un halogène, le groupe hydroxy, un radical alkyle inférieur ou alcoxy inférieur et

$V_1$, $V_2$, $V_3$ et $V_4$ sont chacun l'hydrogène, un radical alkyle inférieur, cycloalkyle en $C_5$–$C_6$ ou le radical benzyle, ou bien les ensembles ($V_1$ et $V_2$) ou ($V_3$ et $V_4$) forment chacun un radical alkylène en $C_3$–$C_6$,

et où les cycles $A_1$, $B_1$ et $D_1$, indépendamment l'un de l'autre, sont chacun non-substitués, ou substitués par un ou deux substituants choisis parmi les radicaux cyano, halogèno, alkyle inférieur, phényle et alcoxy inférieur.

9. Composés bis-quinazoliniques selon la revendication 8, caractérisés en ce que, dans la formule (3), $Y_2$ représente un radical de formule (3a).

10. Composés bis-quinazoliniques selon la revendication 9, caractérisés en ce que, dans la formule (3a), $X_4$ et $X_5$ représentent chacun un radical alkyle inférieur ou le radical benzyle, et $X_6$ l'hydrogène.

11. Composés bis-quinazoliniques selon l'une des revendications 8 à 10, caractérisés en ce que, dans la formule (3), $Q_1$ représente un radical alkylène ou alkylidène ayant chacun au plus 4 atomes de carbone, en particulier le radical isopropylidène ou butylidène.

12. Composés bis-quinazoliniques selon l'une des revendications 8 à 11, caractérisés en ce que, dans la formule (3), le cycle $A_1$ est non-substitué, et les cycles $B_1$ et $D_1$ sont non-substitués ou substitués par le radical méthyle et/ou tert-butyle.

13. Composés bis-quinazoliniques selon la revendication 8, caractérisés en ce que, dans la formule (3), $Y_2$ désigne le radical de formule (3d).

14. Composés bis-quinazoliniques selon l'une des revendications 1 ou 8, caractérisés en ce que, dans les formules (1) et (3), Q et $Q_1$ se trouvent chacun en position para par rapport aux atomes d'oxygène.

15. Composés bis-quinazoliniques selon la revendication 1 ou 8, caractérisés en ce qu'ils correspondent à la formule

(5)

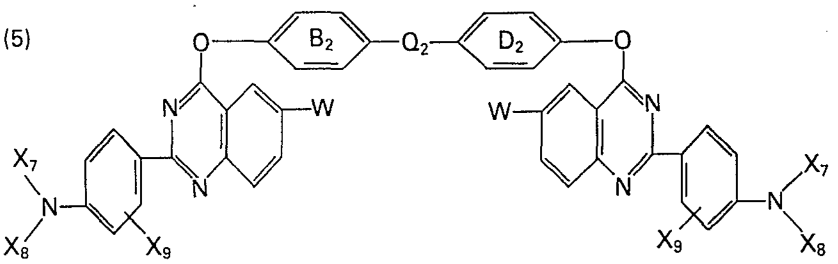

dans laquelle

$Q_2$ est un radical alkylène ou alkylidène à chaîne droite ou ramifiée, ayant chacun au plus 4 atomes de carbone, ou le radical cyclohexylidène,

$X_7$ et $X_8$ sont chacun un radical alkyle inférieur, le radical β-cyanoéthyle, benzyle ou phényle, ou $-NX_7X_8$ est un groupe pipéridino,

$X_9$ est l'hydrogène, le radical méthyle, méthoxy ou éthoxy, et

W est l'hydrogène, un halogène, le radical méthyle ou méthoxy, et

les cycles $B_2$ et $D_2$ sont non-substitués, ou substitués par un ou deux substituants choisis parmi les radicaux méthyle, méthoxy et tert-butyle.

16. Composés bis-quinazoliniques selon l'une des revendications 1 ou 8, caractérisés en ce qu'ils correspondent à la formule

(4)

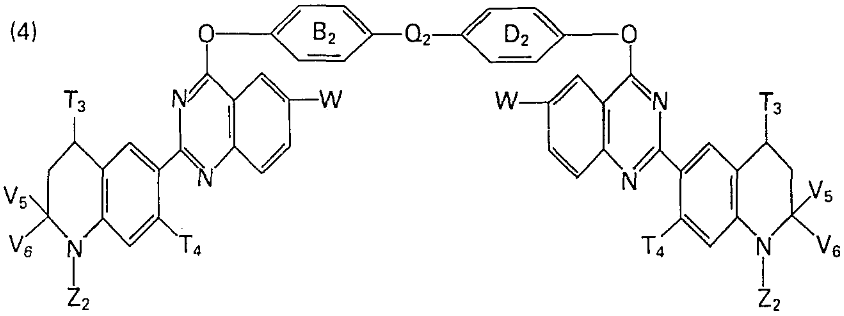

dans laquelle

$Q_2$ est un radical alkylène ou alkylidène à chaîne droite ou ramifiée, ayant chacun au plus 4 atomes de carbone,

$Z_2$ est un radical alkyle ayant de 1 à 8 atomes de carbone, ou le radical β-cyanoéthyle ou benzyle,

$T_3$, $V_5$ et $V_6$ sont chacun un radical alkyle inférieur,

$T_4$ est l'hydrogène ou le radical méthyle, et

W est l'hydrogène, un halogène, le radical méthyle ou méthoxy, et les cycles $B_2$ et $D_2$ sont non-substitués, ou substitués par un ou deux substituants choisis parmi les radicaux méthyle, méthoxy et tert-butyle.

17. Procédé pour la préparation de composés bis-quinazoliniques ayant la formule donnée dans la revendication 1, caractérisé en ce qu'on fait réagir 1 mole d'un composé bisphénolique de formule

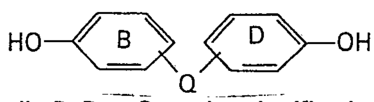

dans laquelle B, D et Q ont les significations donnée dans la revendication 1, sur 2 moles d'un composé 4-halogéno-quinazolinique de formule

(7) 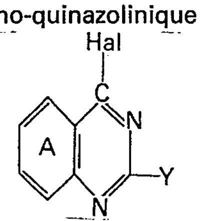

dans laquelle A et Y ont les significations données dans la revendication 1 et Hal désigne un halogène.

18. Matériau d'enregistrement sensible à la pression ou à la chaleur, caractérisé en ce qu'il contient, dans son système de réactifs colorés, en tant que substance chromogène au moins un composé bis-quinazolinique ayant la formule donnée dans l'une des revendications 1 à 16.

19. Matériau d'enregistrement sensible à la pression selon la revendication 18, caractérisé en ce qu'il contient le composé bis-quinazolinique dissous dans un solvant organique, et au moins un accepteur d'électrons solide.

20. Matériau d'enregistrement sensible à la pression selon l'une des revendications 18 et 19, caractérisé en ce que le composé bis-quinazolinique est encapsulé dans des microcapsules.

21. Matériau d'enregistrement sensible à la pression selon la revendication 20, caractérisé en ce que le composé bis-quinazolinique encapsulé se présente sous la forme d'une couche au verso d'une feuille de transfert, et que l'accepteur d'électrons se présente sous la forme d'une couche au recto de la feuille réceptrice.

22. Matériau d'enregistrement sensible à la pression selon l'une des revendications 18 à 21, caractérisé en ce qu'il contient, outre le composé bis-quinazolinique, une ou plusieurs autres substances chromogènes.

23. Matériau d'enregistrement sensible à la chaleur selon la revendication 18, caractérisé en ce qu'il contient, dans au moins une couche, au moins une substance chromogène, un accepteur d'électrons et éventuellement un liant, la substance chromogène ayant la formule donnée dans l'une des revendications 1 à 16.